(19) Europäisches Patentamt
European Patent Office
Office européen des brevets

(11) Publication number: **0 135 330**
**B1**

(12) # EUROPEAN PATENT SPECIFICATION

(45) Date of publication of patent specification: **07.01.88**

(51) Int. Cl.$^4$: **C 07 C 91/12**, C 07 C 89/02

(21) Application number: **84305293.7**

(22) Date of filing: **03.08.84**

(54) **Dehydrohalogenation process for producing polyamino compounds.**

(30) Priority: **12.08.83 JP 147853/83**

(43) Date of publication of application:
**27.03.85 Bulletin 85/13**

(45) Publication of the grant of the patent:
**07.01.88 Bulletin 88/01**

(84) Designated Contracting States:
**DE FR GB IT**

(56) References cited:
**EP-A-0 088 623**
**GB-A-2 100 718**

(73) Proprietor: **MITSUBISHI GAS CHEMICAL COMPANY, INC.**
**5-2, Marunouchi 2-chome Chiyoda-Ku**
**Tokyo (JP)**

(72) Inventor: **Miyamoto, Akira Mitsubishi Gas Chemical Co. Inc.**
**Polymer Research Lab. 6-2 Higashiyahata 5-chome**
**Hiratsuka-shi Kanagawa (JP)**
Inventor: **Seki, Kiichiro Mitsubishi Gas Chemical Co. Inc.**
**Polymer Research Lab. 6-2 Higashiyahata 5-chome**
**Hiratsuka-shi Kanagawa (JP)**
Inventor: **Nishimura, Toshiaki Mitsubishi Gas Chem. Co. Inc.**
**Polymer Research Lab. 6-2 Higashiyahata 5-chome**
**Hiratsuka-shi Kanagawa (JP)**

(74) Representative: **Diamond, Bryan Clive et al**
**Gee & Co. Chancery House Chancery Lane**
**London WC2A 1QU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 135 330 B1

## Description

This invention relates to an improved process for producing a polyamino compound. More particularly, it relates to a process for producing a polyamino compound represented by the formula (I) hereinafter described having a markedly reduced content of alkali metal ions and/or halogen ions and is useful as a hardening agent for epoxy resins.

Polyamino compounds obtained by dehydrohalogenation between xylylenediamine or bisaminomethylcyclohexane (hereinafter referred to as "diamine") and an epihalohydrin in the presence of an alkali, and the use of the polyamino compounds as hardening agent for epoxy resins have already been proposed in Japanese Patent Applications Nos. 36263/82 and 88525/72.

In producing the above-described polyamino compounds, when the diamine is used in a stoichiometrically large excess amount with respect to the epihalohydrin, a large amount of unreacted diamine remains in the reaction product, and such unreacted diamine should be subsequently removed by distillation in order to use the reaction product as a hardening agent for epoxy resins. On the contrary, when the reaction is carried out using 2 mols of the diamine per mol of the epihalohydrin, there is no need to remove the unreacted diamine, but the resulting reaction mixture becomes highly viscous, resulting in difficulty of separating the alkali metal halide formed by the reaction, particularly by filtration or the like.

The alkali used in the dehydrohalogenation reaction between the diamine and the epihalohydrin is generally alkali metal hydroxides, in particular, sodium hydroxide. When the alkali metal hydroxide is used in an amount below a stoichiometrical amount, i.e., less than 1 mol per mol of the epihalohydrin, the dehydrohalogenation reaction would not proceed sufficiently whereby halogen ions remain in the polyamino compound as a final product. On the other hand, when the alkali metal hydroxide is used in an amount over a stoichiometrical amount, the alkali metal hydroxide leaves alkali metal ions in the final polyamino product, thereby causing marked coloration of the product. These halogen ions or alkali metal ions are in no way favorable for the polyamino compounds to be used as hardening agents for epoxy resins. Accordingly, it is important that the reaction for producing polyamino compounds should be performed using a precisely stoichiometrical amount of the alkali metal hydroxide. It is, however, very difficult to control the reaction so as to react a precisely stoichiometrical amount of the alkali metal hydroxide in the production of polyamino compounds on an industrial scale.

A primary object of this invention is to provide an improved process for producing a polyamino compound having a markedly reduced content of halogen ions and/or alkali metal ions.

Another object of this invention is to provide an improved process for producing a polyamino compound by dehydrohalogenation between a diamine and an epihalohydrin in which alkali metal halides can be easily removed from the reaction product.

As a result of extensive studes on a process for producing a polyamino compound represented by the formula (I):

$$H_2N-CH_2-\left(A-CH_2-NH-CH_2-\underset{OH}{\overset{R}{C}}-CH_2-NH-CH_2\right)_n-A-CH_2-NH_2 \qquad (I)$$

wherein A represents a phenylene group or a cyclohexylene group; R represents a hydrogen atom or a methyl group; and n represents an integer of 1 or more, by dehydrohalogenation between a diamine and an epihalohydrin, the present inventors have found that an improved process comprising the following four steps (A) to (D) provides a reaction mixture from which an alkali metal halide can easily be removed, e.g., by filtration and provides a final product having a markedly reduced content of halogen ions and/or alkali metal ions and, thus, devised the present invention. That is, the process comprises:

(A) a step of reacting a diamine and an epihalohydrin in the presence of P mols ($P<1$) of an alkali metal hydroxide per mol of the epihalohydrin;

(B) a step of adding to the reaction mixture obtained in step (A) an alkali metal carbonate in an amount of Q mols, per mol of epihalohydrin wherein Q satisfies the formula:

$$2\times(1-P)\leqq Q\leqq 4\times(1-P)$$

wherein P and Q each represents an amount (in mol) of the alkali metal hydroxide and the alkali metal carbonate, respectively;

(C) a step of separating the reaction mixture obtained in Step (B) into a filtrate and a filter cake; and

(D) a step of removing water from the filtrate obtained in Step (C) by distillation and separating the resulting distillation residue into a precipitated solid and a polyamino compound.

The diamine which is used in the present invention is represented by the formula (II):

$$H_2N-CH_2-A-CH_2-NH_2 \qquad (II)$$

wherein A is as defined above, and includes *m*-xylylenediamine and 1,3-bisaminomethylcyclohexane.

These diamines may be used in a mixture containing up to 40% by weight of *p*-xylylenediamine or 1,4-bisaminomethylcyclohexane.

The epihalohydrin to be reacted with the diamine (II) is represented by the formula (III):

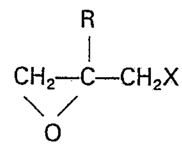

(III)

wherein R is as defined above; and X represents a chlorine atom or a bromine atom, and includes epichlorohydrin, epibromohydrin and β-methylepichlorohydrin.

The present invention will be hereinafter described in greater detail with reference to each step.

Step (A):

An epihalohydrin is added to an excess of diamine in the presence of not more than a stoichiometrical amount of an alkali metal hydroxide to conduct an addition-dehydrohalogenation reaction. The amount of the diamine used in this step is more than 1 mol, preferably 1.5 to 6 mols, per mol of the epihalohydrin. If the amount of the diamine is less than 1.5 mols, gelation of the reaction mixture tends to occur during the reaction. When the diamine is used in an amount more than 6 mols, a large amount of unreacted diamine remains in the final polyamine compound thereby reducing the yield of the product.

The alkali metal hydroxide to be used in Step (A) preferably includes sodium hydroxide and potassium hydroxide in the form of either a solid or an aqueous solution thereof. When the alkali metal hydroxide is employed in a solid form, it is desirable to use a small amount of water. In case of using an aqueous solution of alkali metal hydroxide, it is desirable that the concentration of alkali metal hydroxide is 40% by weight oc more.

The amount of the alkali metal hydroxide (P mol) is less than 1 mol, preferably not less than 0.5 mol, per mol of the epihalohydrin. When it is used in an amount of 1 mol or more, alkali metal ions remain in the final polyamino compound. To the contrary, with less than 0.5 mol of the alkali metal hydroxide, the amount of alkali metal carbonate to be added in the subsequent Step (B) should be unfavorably increased. Since the reaction in Step (A) is exothermic, the reaction is preferably carried out by dropwise adding the epihalohydrin to the diamine. The reaction temperature ranges from about 50°C to about 120°C, preferably from 100°C to 120°C. At temperatures lower than 50°C, the reaction requires a prolonged time, and at temperatures exceeding about 120°C, side reactions of the epihalohydrin tend to occur thereby forming a gelatinous by-product.

Step (B):

In Step (B), the dehydrohalogenation reaction of Step (A) is completed by the addition of an excess amount of alkali metal carbonate.

The alkali metal carbonate used in this step is preferably sodium carbonate and potassium carbonate. The alkali metal carbonate can be used either as a solid or as an aqueous solution, preferably as a solid form. The amount of the alkali metal carbonate Q mol per mol of epihalohydrin) to be added in this step should satisfy the following formula:

$$2\times(1-P)\leqq Q\leqq 4\times(1-P)$$

If Q is greater than $4\times(1-P)$, the amount of the unreacted alkali metal carbonate increases, resulting in undesirable increased volume of the filter cake left in Step (C) or (D). On the other hand, if Q is smaller than $2\times(1-P)$, the dehydrohalogenation reaction becomes insufficient and halogen ions tend to remain in the final polyamino compound.

The reaction temperature is preferably in the range of from 50°C to 90°C. At temperatures exceeding 90°C, separability of the reaction mixture into a filtrate and a filter cake in Steps (C) and (D) becomes poor, and at temperatures lower than 50°C, it takes a long period of time to complete the reaction.

Step (C):

In the step, the reaction mixture obtained through Steps (A) and (B) is separated into a filtrate composed mainly of the desired polyamino compound and water produced during the reaction and a filter cake comprising an alkali metal halide that has been produced in Steps (A) and (B), and the unreacted alkali metal carbonate. The reaction system has a low viscosity because of the presence of water produced and, therefore, the separation into a filtrate and a filter cake can be done effectively. Moreover, in spite of the presence of water in the reaction mixture, more than 90% of the produced alkali metal halide and the unreacted alkali metal carbonate can be removed in this step.

Step (D):

In Step (D), the filtrate obtained in Step (C) is distilled to remove the water contained therein, and the

resulting distillation residue is then filtered to separate the desired polyamino compound from a filter cake comprising the alkali metal halide, etc. which has been precipitated during the distillation. Although the distillation residue after the removal of water is a highly viscous liquid, it can be easily filtered to obtain the polyamino compound as a filtrate because the amount of the cake is relatively very small.

Distillation to remove water is preferably carried out at a temperature not higher than about 120°C under reduced pressure, preferably 1333 to 13332 Pas 10 to 100 mmHg.

Separation in both Step (C) and Step (D) is carried out by a known technique, such as centrifugation, pressure filtration, or filtration under reduced pressure.

The reaction product obtained according to the process as described above is usually a mixture of compounds of the formula (I) wherein *n* is an integer of 1 or more. Preferably, the mixture has an *n* value of 2 or less on average. To this effect, the reaction product may contain compounds of the formula (I) wherein *n* is 2 or more. Further, a small amount of the unreacted diamine may be contained in the reaction product.

The polyamino compounds obtained according to the process of the present invention exhibit particular performances for various uses. For example, when the polyamino compound of this invention is employed as a hardening agent for epoxy resins, it is confirmed that hardening sufficiently occurs at a temperature as low as 0° to 5°C and that the hardened product is much superior in softness as compared with that obtained by using the diamine alone as a hardening agent. Further, use of the diamine hardening agent alone is generally accompanied by a whitening phenomenon of hardened products due to absorption of carbon dioxide in air, whereas the polyamino compounds of this invention do not cause such a whitening phenomenon. Furthermore, when epoxy resins are coated in multi-layer, there are usually involved unavoidable disadvantages, such as separation between layers and reduction in impact resistance of the coated layers. By contrast, it has been found that the epoxy resin hardened with the polyamino compound of this invention is substantially free from these disadvantages.

Further, it has been found that polyamide resins which are prepared by polycondensation of the polyamino compound of this invention as one of diamine components with a dibasic acid component show high softness and improved impact-resistance.

Thus, since the polyamino compounds obtained by the present invention are polyfunctional compounds having two primary amino groups, two or more secondary amino groups and one or more secondary alcohol residues, they exhibit particular properties in various uses. It is also possible to use these polyamino compounds as a reactive starting material for producing high polymers.

The present invention is further illustrated in greater detail by the following examples and comparative examples, but the present invention is not limited thereto. In these examples, all percents are by weight unless otherwise indicated.

Example 1

A four-necked flask equipped with a stirrer, a dropping funnel, a thermometer and a condenser were charged with 1,088 g (8 mols) of *m*-xylylenediamine and 317 g of a 50% aqueous solution of sodium hydroxide (sodium hydroxide content: 3.96 mols). Under stirring in a nitrogen atmosphere, 370 g (4 mols) of epichlorohydrin was added dropwise thereto over a period of 1 hour while maintaining the reaction temperature at 70°C. After completion of the dropwise addition, the temperature of the reaction system was elevated to 100°C where the reaction was further continued for an additional 3 hours. 8.5 grams (0.08 mol) of sodium carbonate was then added to the mixture at a temperature of 80°C, and the resulting mixture was allowed to react for 3 hours. After cooling the mixture to 50°C, the precipitated sodium chloride and sodium carbonate were separated from the reaction mixture by filtration. The filtrate was then subjected to distillation at a temperature of 90 to 100°C under a pressure of about 13332 Pas (100 mmHg) to remove water. Then, the precipitated sodium chloride was removed by filtration from the distillation residue. The filterability of the residue at this time is shown in Table 1. The resulting polyamino product was obtained in a yield of 1,246 g and had an average molecular weight of 320. Physical properties of the product are also shown in Table 1.

Example 2

The same procedures as described in Example 1 were repeated except for using 1,136 g (8 mols) of 1,3-bisaminomethylcyclohexane in place of 1,088 g of m-xylylenediamine.

The product was obtained in a yield of 1,290 g and had an average molecular weight of 330. The filterability of the distillation residue and physical properties of the resulting product are shown in Table 1.

Comparative Example 1

The same reaction vessel as used in Example 1 were charged with 1,088 g (8 mols) of *m*-xylylenediamine and 327 g of a 50% aqueous solution of sodium hydroxide (sodium hydroxide content: 4.08 mols), and 370 g (4 mols) of epichlorohydrin was added dropwise thereto over 1 hour while maintaining the reaction temperature at 60°C. After completion of the dropwise addition, stirring was continued at the same temperature for an additional two hours to effect the reaction. Thereafter, the reaction mixture was subjected to distillation at 90 to 100°C under reduced pressure of about 13332 Pas about 100 mmHg) to remove water, and sodium chloride precipitated in the residue was removed by filtration.

The yield of the product thus obtained was 1.050 g. The filterability of the distillation residue and physical properties of the resulting product are shown in Table 1.

TABLE 1

| | Example 1 | Example 2 | Comparative Example 1 |
|---|---|---|---|
| Viscosity at 25°C (poise) | 150 | 400 | 300 |
| *m*-Xylenediamine Content (wt%) | 30 | 28 | 32 |
| Chlorine Ion Content (ppm) | 100 | 80 | 200 |
| Sodium Ion Content (ppm) | 80 | 90 | 3000 |
| Color (Gardner 1) | 1 or less | 1 or less | 5 |
| Filterability* (hour) | 0.2 | 0.3 | 1.0 |

Note:
*Time required for filtering the bottom using a Nutsche funnel through a filter paper at a mother liquor temperature of 50°C under a pressure reduced by an aspirator.

**Claims**

1. A process for producing a polyamino compound represented by the formula (I):

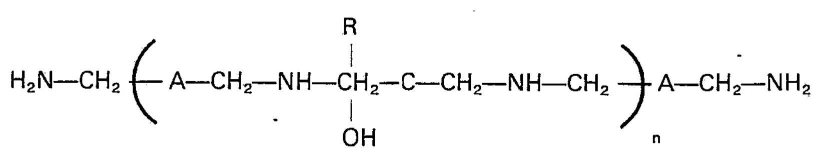
(I)

wherein A represents a phenylene group or a cyclohexylene group; R represents a hydrogen atom or a methyl group; and n represents an integer of 1 or more, comprising dehydrohalogenating a diamine represented by the formula (II):

$$H_2N—CH_2—A—CH_2—NH_2 \quad (II)$$

wherein A is as defined above, with an epihalohydrin represented by the formula (III):

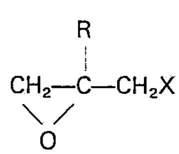
(III)

wherein R is as defined above; and X represents a chlorine atom or a bromine atom, in the presence of an alkali, the characterised in that the process comprises the steps of:

(A) reacting the diamine with the epihalohydrin in the presence of an alkali metal hydroxide in an amount of P mol, wherein P is less than 1, per mol of the epihalohydrin;

(B) adding to the reaction mixture obtained in Step (A) an alkali metal carbonate in an amount of Q mols per mol of epihalohydrin, wherein Q satisfies the following formula:

$$2\times(1-P)\leqq Q\leqq 4\times(1-P)$$

wherein P and Q each represents a number of moles of the alkali metal hydroxide and the alkali metal carbonate, respectively;

(C) separating the reaction mixture obtained in Step (B) into a filtrate and a filter cake; and

(D) removing water from the filtrate obtained in Step (C) by distillation and separating the resulting distillation residues into a precipitated solid and a polyamino compound.

2. A process as claimed in Claim 1, wherein the diamine is used in an amount of more than 1 mol per mol of the epihalohydrin.

3. A process as claimed in Claim 2, wherein the diamine is used in an amount of 1.5 to 6 mols per mol of the epihalohydrin.

4. A process as claimed in Claim 1, 2 or 3, wherein the diamine is *m*-xylylenediamine or 1,3-bisaminomethylcyclohexane.

5. A process as claimed in any of Claims 1 to 4, wherein the epihalohydrin is epichlorohydrin, epibromohydrin or β-methylepichlorohydrin.

6. A process as claimed in any of Claims 1 to 5, wherein the alkali metal hydroxide is present in an amount of not less than 0.5 mol and less than 1 mol per mol of the epihalohydrin.

7. A process as claimed in any of Claims 1 to 6, wherein the alkali metal hydroxide is sodium hydroxide or potassium hydroxide.

8. A process as claimed in any of Claims 1 to 7, wherein the alkali metal hydroxide is a solid or an aqueous solution having a concentration of 40% by weight or more of said alkali metal hydroxide.

9. A process as claimed in any of Claims 1 to 8, wherein the reaction in Step (A) is carried out at a temperature of 50° to 120°C.

10. A process as claimed in Claim 9, wherein the reaction in Step (A) is carried out at a temperature of 100° to 120°C.

11. A process as claimed in any of Claims 1 to 10, wherein the alkali metal carbonate is sodium carbonate or potassium carbonate.

12. A process as claimed in any of Claims 1 to 11, wherein the reaction Step (B) is carried out at a temperature of 50° to 90°C.

13. A process as claimed in any of Claims 1 to 12, wherein the alkali metal carbonate is used in a solid form.

14. A process as claimed in any preceding claim, wherein the separation in Step (C) and Step (D) is carried out by centrifugation, pressure filtration or filtration under reduced pressure.

15. A process as claimed in any preceding claim, wherein the distillation in Step (D) is carried out at a temperature of 120°C or lower under a reduced pressure of 1333 to 13332 Pas (10 to 100 millimetres of mercury).

**Patentansprüche**

1. Verfahren zur Herstellung einer Polyamino-Verbindung der Formel (I)

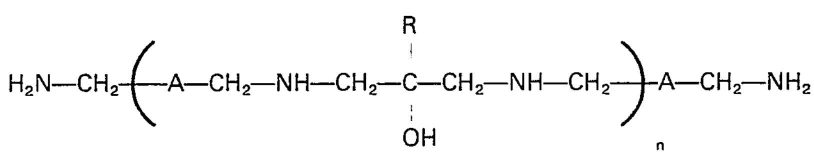

(I)

worin A eine Phenylengruppe oder eine Cyclohexylengruppe bedeutet; R ein Wasserstoffatom oder Methylgruppe bedeutet; und n eine ganze Zahl von 1 oder mehr ist, bei dem man ein Diamin der Formel (II)

$$H_2N—CH_2—A—CH_2—NH_2 \quad (II)$$

worin A die vorher angegebene Bedeutung hat, mit einem Epihalogenhydrin der Formel (III)

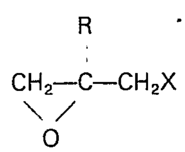

(III)

worin R die vorher angegebene Bedeutung bei und X ein Chloratom oder ein Bromatom bedeutet, in Gegenwart von Alkali dehydrohalogeniert, dadurch gekennzeichnet, dass das Verfahren die folgenden Stufen umfasst:

(A) Umsetzen des Diamins mit dem Epihalogenhydrin in Gegenwart eines Alkalihydroxids in einer Menge von P mol, worin P kleiner als 1 ist, pro Mol des Epihalogenhydrins;

(B) Zugabe zu dem Reaktionsgemisch, erhalten in Stufe (A), eines Alkalicarbonates in einer Menge von Q Molen pro Mol des Epihalogenhydrins, wobei Q der folgenden Formel genügt:

$$2\times(1-P)\leqq Q\leqq 4\times(1-P)$$

worin P und Q jeweils die Anzahl der Mole an Alkalihydroxid bzw. an Alkalicarbonat bedeutetn;

(C) Trennen des in Stufe (B) erhaltenen Reaktionsgemisches in ein Filtrat und einen Filterkuchen; und

(D) Entfernen von Wasser aus dem in Stufe (C) erhaltenen Filtrat durch Destillation und Trennen des erhaltenen Destillationsrückstandes in einen ausgefallenen Feststoff und eine Polyamino-Verbindung.

2. Verfahren gemäss Anspruch 1, bei dem das Diamin in einer Menge von 1 Mol pro Mol des Epihalogenhydrins verwendet wird.

3. Verfahren gemäss Anspruch 2, bei dem das Diamin in einer Menge von 1,5 bis 6 Mol pro Mol des Epihalogenhydrins verwendet wird.

4. Verfahren gemäss Anspruch 1, 2 oder 3, bei dem das Diamin m-Xylylendiamin oder 1,3-Bisaminomethylcyclohexan ist.

5. Verfahren gemäss einem der Ansprüche 1 bis 4, bei dem das Epihalogenhydrin Epichlorhydrin, Epibromhydrin oder β-Methylepichlorhydrin ist.

6. Verfahren gemäss einem der Ansprüche 1 bis 5, bei dem das Alkalihydroxid in einer Menge von nicht weniger als 0,5 Mol und weniger als 1 Mol pro Mol des Epihalogenhydrins vorhanden ist.

7. Verfahren gemäss einem der Ansprüche 1 bis 6, bei dem das Alkalihydroxid Natriumhydroxid oder Kaliumhydroxid ist.

8. Verfahren gemäss einem der Ansprüche 1 bis 7, bei dem das Alkalihydroxid ein Feststoff oder eine wässrige Lösung mit einer Konzentration von 40 Gew.-% oder mehr an Alkalihydroxid ist.

9. Verfahren gemäss einem der Ansprüche 1 bis 8, bei dem man die Reaktion in Stufe (A) bei einer Temperatur von 50 bis 120°C durchführt.

10. Verfahren gemäss Anspruch 9, bei dem man die Reaktion in Stufe (A) bei einer Temperatur von 100 bis 120°C durchführt.

11. Verfahren gemäss einem der Ansprüche 1 bis 10, bei dem das Alkalicarbonat Natriumcarbonat oder Kaliumcarbonat ist.

12. Verfahren gemäss einem der Ansprüche 1 bis 11, bei dem man die Reaktionsstufe (B) bei einer Temperatur von 50 bis 90°C durchführt.

13. Verfharen gemäss einem der Ansprüche 1 bis 12, bei dem man das Alkalicarbonat in fester Form verwendet.

14. Verfahren gemäss einem der vorhergehenden Ansprüche, bei dem die Trennung in Stufen (C) und (D) durch Zentrifugieren, Filtern unter Druck oder Filtern unter vermindertem Druck durchgeführt wird.

15. Verfahren gemäss einem der vorhergehenden Ansprüche, bei dem man die Destillation in Stufe (D) bei einer Temperatur von 120°C oder weniger unter einem verminderten Druck von 1.333 bis 13.332 Pas (10 bis 100 mm Quecksilber) durchführt.

**Revendications**

1. Procédé pour fabriquer un composé polyamino représenté par la formule (I):

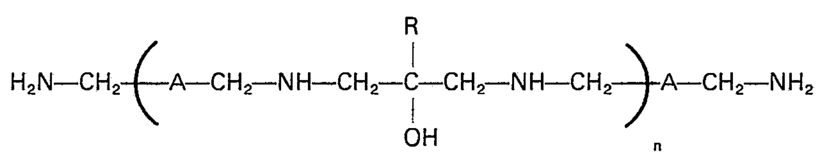

(I)

dans laquelle A représente un groupe phénylène ou un groupe cyclohexylène; R représente un atome d'hydrogène ou un groupe méthyle; n représente un entier égal à 1 ou plus, comprenant la déshydrohalogénation d'une diamine représentée par la formule (II):

$$H_2N{-}CH_2{-}A{-}CH_2{-}NH_2 \qquad (II)$$

dans laquelle A est comme défini ci-dessus avec une épihalohydrine représentée par la formule (III):

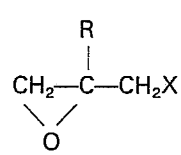

(III)

dans laquelle R est comme défini ci-dessus et X représente un atome de chlore ou de brome, en présence d'un alcali, caractérisé en ce que le procédé comprend les étapes suivantes:

(A) réaction de la diamine avec l'épihalohydrine en présence d'un hydroxyde de métal alcalin en quantité de P mole, où P est inférieur à 1, par mole d'épihalohydrine;

(B) addition au mélange de réaction obtenu dans l'étape (A) d'un carbonate de métal alcalin en quantité de Q moles, par mole d'épihalohydrine, où Q satisfait la relation suivante:

$$2\times(1-P)\leqq Q\leqq 4\times(1-P)$$

dans laquelle P et Q représentent chacun le nombre de moles de l'hydroxyde de métal alcalin et du carbonate de métal alcalin respectivement;

(C) séparation du mélange de réaction obtenu à l'étape (B) en un filtrat et un tourteau de filtration; et

(D) séparation de l'eau du filtrat obtenu à l'étape (C) par distillation et séparation du résidu de distillation résultant en un solide précipité et un composé polyamino.

2. Procédé selon la revendication 1, dans lequel la diamine est utilisée en quantité de plus de 1 mole par mole d'épihalohydrine.

3. Procédé selon la revendication 2, dans lequel la diamine est utilisée en quantité de 1,5 à 6 moles par mole de l'épihalohydrine.

4. Procédé selon la revendication 1, 2 ou 3, dans lequel la diamine est la *m*-xylylènediamine ou le 1,3-bisaminométhylcyclohexane.

5. Procédé selon l'une quelconque des revendications 1 à 4, dans lequel l'épihalohydrine est l'épichlorohydrine, l'épibromhydrine ou la β-méthylépichlorhydrine.

6. Procédé selon l'une quelconque des revendications 1 à 5, dans lequel l'hydroxyde de métal alcalin est présent en quantité de pas moins de 0,5 mole et moins de 1 mole de l'épihalohydrine.

7. Procédé selon l'une quelconque des revendications 1 à 6, dans lequel l'hydroxyde de métal alcalin est l'hydroxyde de sodium ou l'hydroxyde de potassium.

8. Procédé selon l'une quelconque des revendications 1 à 7, dans lequel l'hydroxyde de métal alcalin est un solide ou une solution aqueuse ayant une concentration de 40% en poids ou plus dudit hydroxyde de métal alcalin.

9. Procédé selon l'une quelconque des revendications 1 à 8, dans lequel la réaction dans l'étape (A) est mise en oeuvre à une température de 50 à 120°C.

10. Procédé selon la revendication 9, dans lequel la réaction dans l'étape (A) est mise en oeuvre à une température de 100 à 120°C.

11. Procédé selon l'une quelconque des revendications 1 à 10, dans lequel le carbonate de métal alcalin est le carbonate de sodium ou le carbonate de potassium.

12. Procédé selon l'une quelconque des revendications 1 à 11, dans lequel l'étape de réaction (B) est mise en oeuvre à une température de 50 à 90°C.

13. Procédé selon l'une quelconque des revendications 1 à 12, dans lequel le carbonate de métal alcalin est utilisé sous forme solide.

14. Procédé selon l'une quelconque des revendications précédentes, dans lequel la séparation dans l'étape (C) et l'étape (D) sont mises en oeuvre par centrifugation, filtration sous pression ou filtration sous pression réduite.

15. Procédé selon l'une quelconque des revendications précédentes, dans lequel la distillation dans l'étape (D) est mise en oeuvre à une température de 120°C ou moins sous une pression réduite de 1 333 à 13 332 Pas (10 à 100 mmHg).